(19) Europäisches Patentamt
European Patent Office
Office européen des brevets

(11) Veröffentlichungsnummer: **0 135 758**
**B1**

(12) # EUROPÄISCHE PATENTSCHRIFT

(45) Veröffentlichungstag der Patentschrift: **27.12.90**

(21) Anmeldenummer: **84109454.3**

(22) Anmeldetag: **08.08.84**

(51) Int. Cl.⁵: **C 07 H 3/06,** C 07 H 15/203, C 07 H 9/02, C 12 Q 1/40

(54) **Oligiucosidderivate.**

(30) Priorität: **08.08.83 DE 3328616**

(43) Veröffentlichungstag der Anmeldung:
**03.04.85 Patentblatt 85/14**

(45) Bekanntmachung des Hinweises auf die Patenterteilung:
**27.12.90 Patentblatt 90/52**

(84) Benannte Vertragsstaaten:
**AT BE CH DE FR GB IT LI LU NL SE**

(56) Entgegenhaltungen:
**EP-A-0 171 960**
**DE-A-2 741 192**
**DE-A-2 755 803**
**US-A-4 233 403**
**LIEBIGS ANNALEN DER CHEMIE, Band 11, 1983, Seiten 1845-2054; K. LAESECKE et al.: "Synthese von modifizierten Maltotriosen"**
**"Seminar Paper", 1982(Japanisch mit Engl. Uebersetzung): "Examination of the reactivity of Alpha-Amylase with various substrates by HPLC", M. Matsui et al..**

(73) Patentinhaber: **BOEHRINGER MANNHEIM GMBH**
**Patentabteilung, Abt. E Sandhofer Strasse 112-132 Postfach 31 01 20**
**D-6800 Mannheim 31 Waldhof (DE)**

(72) Erfinder: **Rauscher, Elli, Dr. rer. nat.**
**Guardinistrasse 71**
**D-8000 München 70 (DE)**
Erfinder: **Schaich, Eugen, Dr. rer. nat.**
**Deglergasse 6**
**D-8120 Weilheim (DE)**
Erfinder: **Neumann, Ulrich, Dr. rer. nat.**
**Drosselweg 2**
**D-8123 Peissenberg/Obb. (DE)**
Erfinder: **Wahlefeld, August, Dr. rer. nat.**
**Alpenblickstrasse 25**
**D-8126 Hohenpeissenberg (DE)**
Erfinder: **Gruber, Wolfgang, Dr. phil. nat.**
**Am Oberanger 7**
**D-8132 Tutzing-Unterzeismering (DE)**
Erfinder: **Empl, Bernhard**
**Am Schleiferhäusl 13**
**D-8120 Weilheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 135 758 B1

(56) Entgegenhaltungen:
"Methods in Carbohydrate Chemistry", Band 2, (1963), Seiten 3-7.

Carbohydrate Research, Band 38 (1974), S.346-351

Carbohydrate Research, Band 108 (1982), S. 97-101

Carbohydrate Research, Band 44 (1975), S. 1-11
Journal of Biochemistry, Band 93 (1983), S. 1055-1060

Dissertation, K. Laeseke, Universität Konstanz, 1982

"dtv-Atlas zur Chemie", Band 2, (1983), S.351
"Organische Chemie", M. Braun und F. Krieger, (1982), S. 122

Bulletin of the Chemical Society of Japan, Band 46, (1973), S. 656-657.

"Römpps Chemie Lexikon" (1966), S.3322 und 3324.

"Römpps Chemie Lexikon" (1973), S. 1806 und 1809.

"The new Encyclopeadia Britannica", Macropaedia, (1981), Band 3, Seiten 823, 832.

(74) Vertreter: Weickmann, Heinrich, Dipl.-Ing. et al
Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820
D-8000 München 86 (DE)

**Beschreibung**

Die Erfindung betrifft neue Verbindungen der in den Ansprüchen angegebenen allgemeinen Formel, ihre Herstellung sowie ihre Verwendung als Substrat für die Bestimmung der α-Amylase.

Die Bestimmung des α-Amylase-Spiegels im Serum ist ein wichtiger klinischer Parameter für die Bauchspeicheldrüsenfunktion. Die handelsüblichen Reagenzien zur Bestimmung der α-Amylase war früher überwiegend auf die Verwendung von Stärke oder Stärkederivaten als Substrate abgestellt. Diese Substrate erwiesen sich jedoch insbesondere hinsichtlich der Einheitlichkeit als unbefriedigend. Um diesen Mangel zu beseitigen, wurden daher Stärke und Stärkederivate durch Oligosaccharide und optisch bestimmbar Derivate derselben ersetzt, wobei insbesondere die Maltotetraose, -pentaose, -hexaoase und -heptatose und deren Derivate interessante Verbesserungen lieferten (DE—OS 27 41 192, DE—OS 27 55 803, US—PS 3 879 263, US—PS 4 000 042).

Eine besonders interessante Ausführungsform der α-Amylase-Bestimmung unter Verwendung der genannten Oligoglucoside ergab sich in Gegenwart von α-Glucosidase, da hierbei ein vollständiger Abbau des Oligoglucosids zu Glucose erreicht werden konnte und die Glucose dann nach den hierfür bekannten Verfahren leicht bestimmt werden konnte (vgl. DE—OS 27 41 192).

Es hat sich jedoch herausgestellt, daß das Hilfsenzym α-Glucosidase die Lagerungsdauer des fertigen Reagenzgemisches verringert, da es auch ohne Einwirkung der α-Amylase eine gewisse Spaltung des Oligoglucosides hervorruft.

Der Erfindung liegt daher die Aufgabe zugrunde, diesen Nachteil zu beseitigen und ein α-Amylasesubstrat zu schaffen, welches auch in Gegenwart von α-Glucosidase ausreichend lagerfähig ist und die Richtigkeit des α-Amylasenachweises verbessert.

Gelöst wird diese Aufgabe erfindungsgemäß durch Verbindungen der allgemeinen Formel I

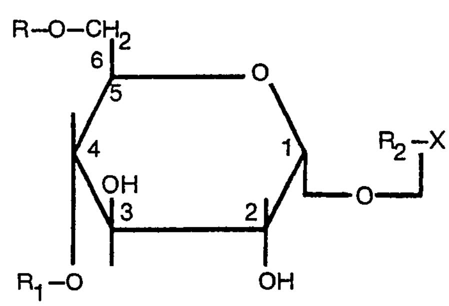

in der R und $R_1$ unabhängig voneinander je eine geradkettige oder verzweigte Alkyl- oder Alkoylgruppe mit 1 bis 6 Kohlenstoffatomen oder einen Phenylrest darstellen, wobei R und $R_1$ auch zusammen eine Methylenbrücke bilden können, deren Wasserstoffatome unabhängig voneinander durch je eine Alkylgruppe mit 1 bis 5 Kohlenstoffatomen oder eine Phenylgruppe substituiert sein können, $R_2$ einen Oligoglucosidrest mit 2 bis 7 Glucoseeinheiten und X ein Wasserstoffatom oder eine Nitrophenylgruppe darstellen.

Es hat sich gezeigt, daß mit den erfindungsgemäßen Verbindungen das gebrauchsfertige Reagenz auch in Gegenwart von α-Glucosidase keinen Veränderungen unterliegt und daher auch nach längerer Dauer richtige α-Amylasewerte liefert.

In der Dissertation K. Laesecke, Universität Konstanz, 1982, wird die Verbindung Phenyl D-(4,6-O-benzyliden-α-D-glucopyranosyl)-(1→4)-D-(α-D-(α-D-glucopyranosyl)-(1→4)-α-D-glucopyranosid beschrieben als Zwischenprodukt im Rahmen der Perbenzylierung des Trisaccharides. Diese Dissertation befaßt sich jedoch weder mit einer α-Amylasebestimmung, noch lassen sich daraus Hinweise entnehmen, wonach durch die beschriebene Überbrückung der 4″—OH und 6″—OH-Gruppen das Problem der Destabilisierung durch das Hilfsenzym α-Glucosidase im Rahmen der α-Amylasebestimmung gelöst werden könnte.

Als Alkylgruppe sind die Methyl-, Äthyl-, Propyl-, Isopropyl-, Butyl-, Isobutyl-, t-Butyl-, n-Pentyl-, deren Isomere, n-Hexyl-, die Isomeren davon sowie die Cyclohexylgruppe zu nennen. Ebenso kommen die den genannten Alkylgruppen entsprechenden Alkoylgruppen als Substituenten an den Sauerstoffatomen in Position 4 und/oder 6 des endständigen Glucosidrestes in Frage. Bevorzugt werden im Rahmen der Erfindung diejenigen Verbindungen, in denen R und $R_1$ zusammen eine gegebenenfalls substituierte Methylenbrücke bilden, besonders bevorzugt solche, welche durch eine Alkylgruppe oder eine Phenylgruppe substituiert sind, insbesondere eine Ethyliden-, oder Benzylidengruppe.

Unter den Oligoglucosidresten $R_2$ werden diejenigen mit 3, 4 und 6 Glucoseeinheiten bevorzugt.

Für X kommen Nitrogruppen-haltige Phenylreste, wie bevorzug die Nitrophenyl- oder die 3,4-Dinitrophenylgruppen in Frage.

Die Herstellung der erfindungsgemäßen Verbindungen kann erfolgen ausgehend von Oligoglucosiden mit 3 bis 8 Glucoseeinheiten, die endständig gegebenenfalls die Gruppe X tragen, indem man diese unter Veresterungs- oder Verätherungsbedingungen mit einer Orthooxyverbindung, vorzugsweise mit

Dialkoxyethan oder dem entsprechenden Benzylderivat umsetzt unter Bildung einer Verbindung der allgemeinen Formel I, in welcher R und $R_1$ zusammen eine gegebenenfalls substituierte Methylengruppe bilden und gegebenenfalls anschließend die frein Oh-Gruppen blockiert, beispielsweise durch Peracetylierung, die Methylenbrücke am Sauerstoffatom in Stellung 4 oder 6 spaltet und erforderlichenfalls die so gebildete freie OH-Gruppe in Stellung 4 oder 6 nochmals veräthert oder verestert bzw. umäthert oder umestert und abschließend die übrigen OH-Blockierungsgruppen, z.B. Acetylgruppen, abspaltet.

Die bei dieser Synthese als Zwischenprodukte auftretenden, im übrigen aber auch als Endprodukte bevorzugten Verbindungen mit einer gegebenenfalls substituierten Methylenbrücke werden erfindungsgemäß hergestellt, indem man eine Verbindung der allgemeinen Formel II

$$R_3—X$$

in der X die vorstehend angegebene Bedeutung hat und $R_3$ ein Oligoglucosidrest mit 3 bis 8 Glucoseeinheiten ist, in Gegenwart von p-Toluolsulfonsäure mit einer Verbindung der allgemeinen Formel III

$$R_4—\overset{R_5}{\overset{|}{C}}—(\text{O-niedrigalkyl})_2$$

in der $R_4$ und $R_5$ unabhängig voneinander je ein H-Atom oder eine Alkylgruppe mit 1 bis 5 Kohlenstoffatomen oder eine Phenylgruppe bedeuten, in einem polaren organischen Lösungsmittel umsetzt.

Als polares Lösungsmittel werden Dimethylformamid oder Formamid bevorzugt, es eignen sich jedoch auch andere polare organische Lösungsmittel mit vergleichbarer Basizität.

Die genannte Umsetzung führt überraschenderweise zu einheitlichen Produkten ohne daß die vorhandenen zahlreichen OH-Gruppen geschützt werden müssen. Eventuell gebildete Nebenprodukte lassen sich unschwierig abtrennen.

Die Umsetzung wird zweckmäßig bei Temperaturen zwischen etwa 10 und etwa 70°C durchgeführt, bevorzugt wird Raumtemperatur. Da bei der Umsetzung kaum Nebenprodukte gebildet werden, arbeitet man zur Erzielung bester Ausbeuten vorzugsweise mit einem stöchiometrischen Überschuß an der Verbindung der allgemeinen Formel III.

Beispiele für Verbindungen der allgemeinen Formel III sind Dimethoxymethan, Dimethoxyäthan, Diäthoxyäthan, Dipropoxyäthan, Dibutoxyäthan, Dimethoxypropan, Dimethoxyisopropan und Phenyldimethoxymethan.

Beispiele für Verbindungen der allgemeinen Formel II sind Maltotetraose, Maltopentaose, Maltohexaose, Maltoheptaose und deren endständig durch eine Nitrophenyl-Gruppe substituierte Derivate wie Mononitrophenyl-Maltoheptaose, 3,4-Dinitrophenyl-Maltoheptaose usw.

Die überlegene Lagerfähigkeit der erfindungsgemäßen Verbindungen zeigt sich darin, daß unter den Bedingungen eines weit verbreiteten, handelsüblichen α-Amylasefarbtests mit p-Nitrophenylmaltoheptaosid als Substrat ($G_7pNP$) nämlich: Puffer pH 7,1; NaCl 50 mM; ca. 30 U/ml α-Glucosidase; 5 mM Substrat bei praktisch identischen Reaktionabläufen hinsichtlich Lag-Phase und Linearität mit 4,6-Ethyliden-p-nitrophenylmaltoheptaosid (Eth-$G_7pNP$) innerhalb von 4 Tagen bei 25°C praktisch keine Glucose gebildet wird, während mit dem $G_7pNP$ eine merkliche Spaltung zu Glucose stattfindet. Unter denselben Bedingungen bei 4°C ergab sich mit dem erfindungsgemaßen Substrat nach 8 Tagen keine Glucosebildung, beim bekannten Vergleichssubstrat hingegen eine deutliche Spaltung. Erfindungsgemäß werden daher neue Verbindungen geschaffen, die als substrat für die α-Amylase in Gegenwart von α-Glucosidase überlegene Eigenschaften aufweisen.

Die folgenden Beispiel erläutern die Erfindung.

### Beispiel 1
### Verfahren zur Herstellung von 4,6-Ethyliden-4-Nitrophenyl-α-D-Maltoheptaosid

Ansatz:

250 g (196 mMol) 4-Nitrophenyl-α-D-Maltoheptaosid
31,5 ml (297 mMol) Acetaldehyd-Dimethylacetal
20 g para-Toluolsulofonsäure·$H_2O$
1,5 l DMF (Dimethylformamid)

Synthese:

250 g 4-Nitrophenyl-α-D-Maltoheptaosid und 20 g para-Toluolsulfonsäure·$H_2O$ werden in ca. 1,5 l DMF gelöst und zur Absolutierung am Rotationsverdampfer zur Trocknung einrotiert. Dabei sinkt der Wassergehalt von 0,4% auf 0,02%.

Der Rückstand wird in 1,5 l DMF gelöst, 31,5 ml Acetaldehyd-Dimethylacetal werden zugegeben und zuerst bei 50°C 9 Stunden lang gerührt, dann noch ca. 10 Stunden bei Raumtemperatur gehalten. Die Reaktionsmischung wird zur Trockene eingeengt, der Rückstand in Wasser gelöst, mit Lithiumhydroxydlösung auf pH 7,3 eingestellt, klarfiltriert, und auf 750 ml eingeengt.

Die HPLC-Analyse ergibt einen Gehalt von ca. 75 bis 80% Ethyliden-4-Nitrophenyl-α-D-Maltoheptaosid mit ca. 20% Ausgangsmaterial.

Chromatographische Reinigung:

Die Probelösung (750 ml) wird auf eine 150 × 25 cm-Säule mit 70 l Dowex 50 WX2 (200 bis 400 mesh) Lithium$^+$ aufgezogen und mit Wasser eluiert bei einem Fluß von 1,5 l/h. Dabei werden Fraktionen a 4,5 l gesammelt und die Chromatographie mittels eines UV-Detektors bei 280 nm verfolgt.

Nach ca. 100 l eluiert nicht umgesetztes 4-nitrophenyl-α-D-Maltoheptaosid, nach ca. 200 l eluiert das gesuchte Prudukt.

Die Fraktionen, die das Produkt enthalten, werden vereinigt und zur Trockene eingeengt. Der Rückstand wird in 1,5 l Methanol gelöst, filtriert und bei 0°C mit 4 l Isopropanol und 10 l Petrolether gefällt. Nach Rühren über Nacht bei 4°C wird das Produkt abgesaugt, mit Isopropanol und Petrolether gewaschen und bei 30°C im Trockenschrank getrocknet.

Ausbeute: 150 g (58% der Theorie), farbloses Pulver, Molekulargewicht 1300.
$H_2O$ (nach K. Fischer) 4,5%
Isopropanol (gaschromatographisch) 5%
Acetaldehyd nach saurer Hydrolyse (enzym.) 91%
$[\alpha]_D$ 25°C, bezogen auf Trockensubstanz = 77° (c = 1, $H_2O$)
HPLC: 99 Flächenprozent (5 μ -$NH_2$-Säule; Acetonitril/Wasser 1:1, 1 mMol/l c-Phosphorsäure, Detektion bei 305 nm).

Beispiel 2
Verfahren zur Herstellung von 4,6-Ethyliden-Malotoheptaose

Ansatz:
10 g (8,7 mMol) Maltoheptaose
1,8 ml (17 mMol) Acetaldehyd-Dimethylacetal
1 g para-Toluolsulfonsäure·$H_2O$
75 ml DMF

Synthese:

10 g Maltoheptaose und 1 g para-Toluolsulfonsäure·$H_2O$ werden in 100 ml DMF gelöst und zur Trockene eingeengt. Auf diese Weise wird der Rückstand wasserfrei gemacht. Der Rückstand vird in 75 ml DMF gelöst, mit 1,7 ml Acetaldehyd-Dimethylacetal versetzt und 15 Stunden verschlossen bei 50°C gerührt. Dann wird die Reaktionslösung zur Trockene eingeengt, der Rückstand im Wasser gelöst, mit Lithiumhydroxyd auf pH 7 neutralisiert, klarfiltriert und auf 50 ml konzentriert.

Chromatographische Reinigung:

Die 50 ml Probenlösungen werden auf eine Chromatographiesäule (180 × 5 cm) mit 3,5 l Dowex 50 WX2 (200 bis 400 mesh) Li$^+$ aufgezogen, eluiert mit Wasser bei einer Fließgeschwindigkeit von 150 ml/h. Es werden 30 ml-Fraktionen gesammelt, die durch einen UV-Detektor (280 nm) laufen.

Nach 1,8 l eluiert Maltoheptaose, nach 2,25 l eluiert Ethyliden-Maltoheptaose.

Die Hauptfraktionen werden vereinigt, zur Trockene einrotiert, in 200 ml Methanol gelöst unter Zugabe von etwas Wasser, mit 200 ml Isopropanol versetzt und bei 4°C gerührt. Dabei fällt das Produkt aus, dieses wird abgesaugt, mit Isopropanol und Petroläther gewaschen und im Vakuum bei 25°C über $P_2O_5$ getrocknet.

Ausbeute: 6,2 g (60% d.Theorie)
Analytik: Wasser (nach K. Fischer) 7,6%
Acetaldehyd nach saurer Hydrolyse (enzym.) 91,2%
$[\alpha]_D$ 25°C, bezogen auf Trockensubstanz (c = 1, $H_2O$) = 69°C
HPLC (Bedingungen siehe Beispiel 1): 96 Flächenprozent.
Die reaktion mit Tetrazoliumblau beweist, daß das reduzierende Ende frei ist.

Beispiel 3

Reagenz: 682,5 mg Ethyliden-$G_7$PNP (5.25 mmol/l) wurden in 100 ml Natriumphosphatpuffer (105 mmol/l), enthaltend Natriumchlorid (52,5 mmol/l), sowie alpha-Glucosidase (42 U/ml) gelöst und auf pH 7,10 eingestellt.

Endkonzentration im Test: Phosphatpuffer 100 mmol/l, NaCl 50 mmol/l, alpha-Glucosidase 40 U/ml.

Testansatz: Zu 2,0 ml auf 25°C temperiertem Reagenz wurden 0,1 ml Probe zugegeben und das Gemisch auf 25°C temperiert. Nach einer Vorinkubationszeit von 4 Minuten (lag phase) wurde die Extinktionszunahme bei Hg 405 nm an einem Eppendorf-Photometer mit Schreiber registriert. Aus der Extinktionsänderung pro Minute (ΔE/min) wurde die Aktivität der Amylase in den Proben nach folgender Formel berechnet:

$$\frac{\Delta E/\text{min} \cdot V \cdot 1000 \cdot 3}{E \cdot v \cdot d} = \Delta E/\text{min} \cdot 7000 \text{ [U/l]}$$

Wiederfindung von alpha-Amylase: Die Bestimmung der Aktivität wurde mit bei 4°C und bei 25°C gelagertem Reagenz in bestimmten Zeitabständen wiederholt und folgende Werte gefunden:

| Lagerung des Reagenz | Humanserum 1 U/1 | Humanserum 2 U/1 | Kontrollserum PNU 572 U/1 |
|---|---|---|---|
| Anfangswert | 118 | 383 | 214 |
| 4 Stunden bei 25°C | 120 | 379 | 214 |
| 8 " " | 120 | 391 | 216 |
| 24 " | 113 | 360 | 209 |
| 32 " | 120 | 392 | 217 |
| 48 " | 126 | 382 | 214 |
| 8 Stunden bei 4°C | 119 | 370 | 210 |
| 24 " | 118 | 387 | 215 |
| 48 " | 123 | 393 | 208 |
| 72 " | 113 | 401 | 223 |
| 80 " | 121 | 375 | 219 |
| Mittelwert/VK | 119 3,2% | 383 3,0% | 214 2,0% |

Die einzelnen Werte liegen in der üblichen Schwankungsbreite für manuelle Bestimmungen von Enzymaktivitäten.

**Patentansprüche für die Vertragsstaaten: BE CH LI DE FR GB IT LU NL SE**

1. Verbindungen der allgemeinen Formel I

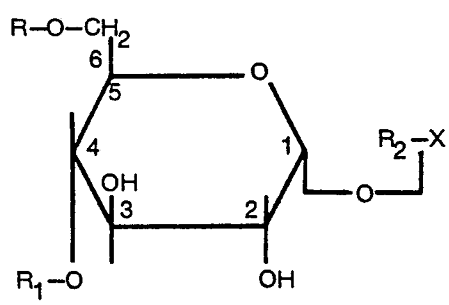

in der R und $R_1$ unabhängig voneinander je eine geradkettige oderverzweigte Alkyl- oder Alkoylgruppe mit 1 bis 6 Kohlenstoffatomen oder einen Phenylrest darstellen, wobei R und $R_1$ auch zusammen eine Methylenbrücke bilden können, deren Wasserstoffatome unabhängig voneinander durch je eine Alkylgruppe mit 1 bis 5 Kohlenstoffatomen oder eine Phenylgruppe substituiert sein können, $R_2$ einen Oligoglucosidrest mit 2 bis 7 Glucoseeinheiten und X ein Wasserstoffatom oder ein Nitrogruppe-haltige Phenylrest darstellen.

2. Verbindung nach Anspruch 1, dadurch gekennzeichnet, daß das R und $R_1$ zusammen eine Ethylidenbrücke bilden.

3. Verfahren zur Herstellung einer Verbindung gemäß Anspruch 1 oder 2, dadurch gekennzeichnet, daß man eine Verbindung der allgemeinen Formel II

$$R_3\text{—}X$$

in der X die in Anspruch 1 angegebene Bedeutung hat und $R_3$ ein Oligoglucosidrest mit 3 bis 8 Glucoseeinheiten ist, in Gegenwart von p-Toluolsulfonsäure mit einer Verbindung der allgemeinen Formel III

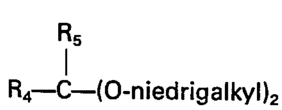

in der $R_4$ und $R_5$ unabhängig voneinander je ein Wasserstoffatom oder eine Alkylgruppe mit 1 bis 5 Kohlenstoffatomen oder eine Phenylgruppe bedeuten, in einem polaren organischen Lösungsmittel umsetzt.

4. Verfahren nach Anspruch 3, dadurch gekennzeichnet, daß man als organisches Lösungsmittel Dimethylformamid verwendet.

5. Verfahren nach Anspruch 3 oder 4, dadurch gekennzeichnet, daß man die Umsetzung zwischen 10°C und 70°C durchführt.

6. Verfahren nach einem der Ansprüche 3 bis 5, dadurch gekennzeichnet, daß man die Verbindung der allgemeinen Formel III im Überschuß einsetzt.

7. Verwendung einer Verbindung gemäß der allgemeinen Formel

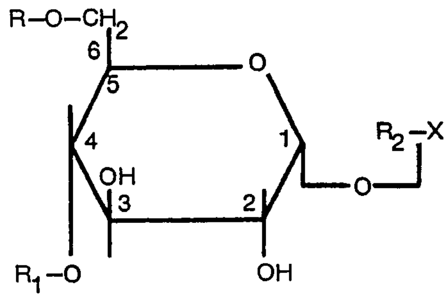

in der R und $R_1$ unabhängig voneinander je eine geradkettige oder verzweigte Alkyl- oder Alkoylgruppe mit 1 bis 6 Kohlenstoffatomen oder einen Phenylrest darstellen, wobei R und $R_1$ auch zusammen eine Methylenbrücke bilden können, deren Wasserstoffatome unabhängig voneinander durch je eine Alkylgruppe mit 1 bis 5 Kohlenstoffatomen oder eine Phenylgruppe substituiert sein können, $R_2$ einen Oligoglucosidrest mit 2 bis 7 Glucoseeinheiten und X ein Wasserstoffatom oder einen optisch bestimmbaren Rest, insbesondere eine Nitrophenylgruppe darstellen, als Substrat bei der Bestimmung von α-Amylase in Gegenwart von α-Glucosidase.

8. Verwendung einer Verbindung gemäß Anspruch 2 als Substrat bei der Bestimmung von α-Amylase in Gegenwart von α-Glucosidase.

**Patentansprüche für den Vertragsstaat: AT**

1. Verfahren zur Herstellung einer Verbindung der allgemeinen Formel I

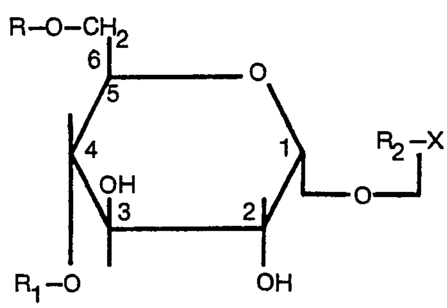

in der R und $R_1$ unabhängig voneinander je eine geradkettige oderverzweigte Alkyl- oder Alkoylgruppe mit 1 bis 6 Kohlenstoffatomen oder einen Phenylrest darstellen, wobei R und $R_1$ auch zusammen eine Methylenbrücke bilden können, deren Wasserstoffatome unabhängig voneinander durch je eine Alkylgruppe mit 1 bis 5 Kohlenstoffatomen oder eine Phenylgruppe substituiert sein können, $R_2$ einen Oligoglucosidrest mit 2 bis 7 Glucoseeinheiten und X ein Wasserstoffatom oder ein Nitrogruppe haltige phenylbiest darstellen, dadurch gekennzeichnet, daß man eine Verbindung der allgemeinen Formel II

$$R_3—X$$

in der X die oben angegebene Bedeutung hat und $R_3$ ein Oligoglucosidrest mit 3 bis 8 Glucoseeinheiten ist, in Gegenwart von p-Toluolsulfonsäure mit einer Verbindung der allgemeinen Formel III

$$R_4—\overset{\displaystyle R_5}{\overset{|}{C}}—(\text{O-niedrigalkyl})_2$$

in der $R_4$ und $R_5$ unabhängig voneinander je ein Wasserstoffatom oder eine Alkylgruppe mit 1 bis 5

Kohlenstoffatomen oder eine Phenylgruppe bedeuten, in einem polaren organischen Lösungsmittel umsetzt.

2. Verfahren nach Anspruch 1, dadurch gekennzeichnet, daß R und $R_1$ zusammen eine Ethylidenbrücke bilden.

3. Verfahren nach Anspruch 1 oder 2, dadurch gekennzeichnet, daß man als organisches Lösungsmittel Dimethylformamid verwendet.

4. Verfahren nach Anspruch 1, 2 oder 3, dadurch gekennzeichnet, daß man die Umsetzung zwischen 10°C und 70°C durchführt.

5. Verfahren nach einem der Ansprüche 1 bis 4, dadurch gekennzeichnet, daß man die Verbindung der allgemeinen Formel III im Überschuß einsetzt.

6. Verwendung einer Verbindung gemäß Ansprüch 1 oder 2 als Substrat bei der Bestimmung von α-Amylase in Gegenwart von α-Glucosidase.

7. Verwendung einer Verbindung gemäß der allgemeinen Formel

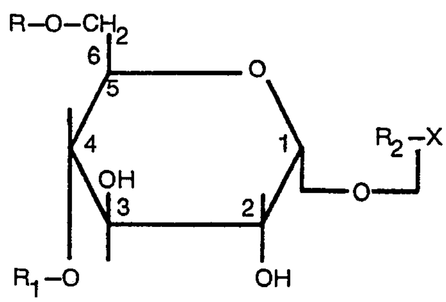

in der R und $R_1$ unabhängig voneinander je eine geradkettige oder verzweigte Alkyl- oder Alkoylgruppe mit 1 bis 6 Kohlenstoffatomen oder einen Phenylrest darstellen, wobei R und $R_1$ auch zusammen eine Methylenbrücke bilden können, deren Wasserstoffatome unabhängig voneinander durch je eine Alkylgruppe mit 1 bis 5 Kohlenstoffatomen oder eine Phenylgruppe substituiert sein können, $R_2$ einen Oligoglucosidrest mit 2 bis 7 Glucoseeinheiten und X ein Wasserstoffatom oder einen optisch bestimmbaren Rest, insbesondere eine Nitrophenylgruppe darstellen, als Substrat bei der Bestimmung von α-Amylase in Gegenwart von α-Glucosidase.

8. Verwendung einer Verbindung gemäß Anspruch 2 als Substrat bei der Bestimmung von α-Amylase in Gegenwart von α-Glucosidase.

**Revendications pour les Etats Contractants: BE CH LI DE FR GB IT LU NL SE**

1. Composés de formule générale I

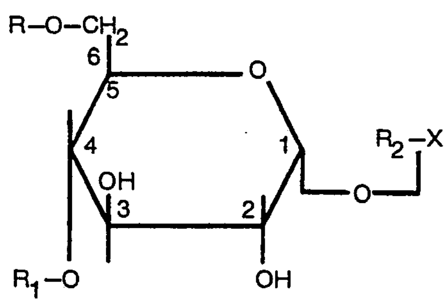

dans laquelle R et $R_1$, indépendamment l'un de l'autre, représentent chacun un groupe alcoyle ou alcanoyle, rectiligne ou ramifié, avec 1 à 6 atomes de carbone ou un radical phényle, R et $R_1$ pouvant également former ensemble un pont méthylène dont les atomes de carbone, indépendamment l'un de l'autre, peuvent être chacun substitué par un groupe alcoyle avec 1 à 5 atomes de carbone ou un groupe phényle, $R_2$ représente un radical oligoglucoside avec 2 à 7 unités glucose et X représente un atome d'hydrogène ou un radical phényle contenant un groupe nitro.

2. Composé selon la revendication 1, caractérisé en ce que R et $R_1$ forment ensemble un pont éthylidène.

3. Procédé pour la preparation d'un composé selon la revendication 1 ou 2, caractérisé en ce qu'on fait réagir un composé de formule générale II

$$R_3—X$$

dans laquelle X a la signification indiquée à la revendication 1 et $R_3$ est un radical oligoglucoside avec 3 à 8 unités glucose, en présence d'acide p-toluènesulfonique, avec un composé de formule générale III

$R_5$
|
$R_4$—C—(O-alcoyl inférieur)$_2$

dans laquelle $R_4$ et $R_5$, indépendamment l'un de l'autre, représentent chacun un atome d'hydrogène ou un groupe alcoyle avec 1 à 5 atomes de carbone ou un groupe phényle, dans un solvant organique polaire.

4. Procédé selon la revendication 3, caractérisé en ce que l'on utilise, comme solvant organique, du diméthylformamide.

5. Procédé selon la revendication 3 ou 4, caractérisé en ce qu'on effectue la réaction entre 10°C et 70°C.

6. Procédé selon l'une des revendications 3 à 5, caractérisé en ce qu'on met en oeuvre le composé de formule générale III en excès.

7. Utilisation d'un composé selon la formule générale

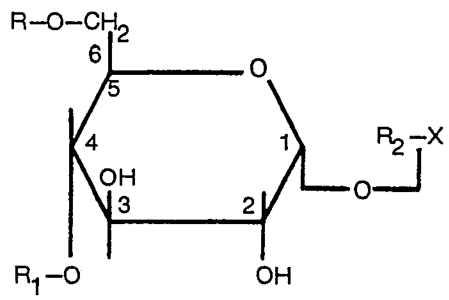

dans laquelle R et $R_1$, indépendamment l'un de l'autre, représentent chacun un groupe alcoyle ou alcanoyle, rectiligne ou ramifié, avec 1 à 6 atomes de carbone ou un radical phényle, R et $R_1$ pouvant également former ensemble un pont méthylène dont les atomes de carbone, indépendamment l'un de l'autre, peuvent être chacun substitué par un groupe alcoyle avec 1 à 5 atomes de carbone ou un groupe phényle, $R_2$ représente un radical oligoglucoside avec 2 à 7 unités glucose et X représente un atome d'hydrogène ou un radical déterminable optiquement, en particulier un groupe nitrophényle, comme substrat dans la détermination de l'α-amylase en présence d'α-glucosidase.

8. Utilisation d'un composé selon la revendication 2 comme substrat dans la détermination de l'α-amylase en présence d'α-glucosidase.

**Revendications pour l'Etat Contractant: AT**

1. Procédé pour la préparation d'un composé de formule générale I

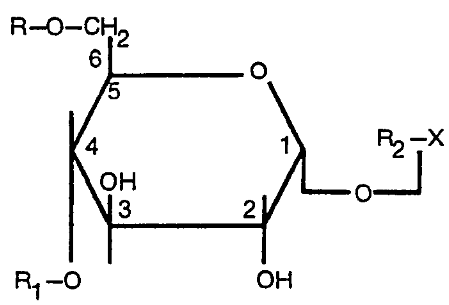

dans laquelle R et $R_1$, indépendamment l'un de l'autre, représentent chacun un groupe alcoyle ou alcanoyle, rectiligne ou ramifié, avec 1 à 6 atomes de carbone ou un radical phényle, R et $R_1$ pouvant également former ensemble un pont méthylène dont les atomes de carbone, indépendamment l'un de l'autre, peuvent être chacun substitué par un groupe alcoyle avec 1 à 5 atomes de carbone ou un groupe phényle, $R_2$ représente un radical oligoglucoside avec 2 à 7 unités glucose et X représente un atome d'hydrogène ou un radical phényle contenant un groupe nitro, caractérisé en ce qu'on fait réagir un composé de formule générale II

$R_3$—X

dans laquelle X a la signification indiquée plus haut et $R_3$ est un radical oligoglucoside avec 3 à 8 unités glucose, en présence d'acide p-toluènesulfonique, avec un composé de formule générale III

$R_5$
|
$R_4$—C—(O-alcoyl inférieur)$_2$

dans laquelle $R_4$ et $R_5$, indépendamment l'un de l'autre, représentent chacun un atome d'hydrogène ou un group alcoyle avec 1 à 5 atomes de carbone ou un groupe phényle, dans un solvant organique polaire.

2. Procédé selon la revendication 1, caractérisé en ce que R et $R_1$ forment ensemble un pont éthylidène.

3. Procédé selon la revendication 1 ou 2, caractérisé en ce qu'on utilise, comme solvant organique, du diméthylformamide.

4. Procédé selon la revendication 1, 2 ou 3, caractérisé en ce qu'on effectue la réaction entre 10°C et 70°C.

5. Procédé selon l'une des revendications 1 à 4, caractérisé en ce qu'on met en oeuvre le composé de formule générale III en excès.

6. Utilisation d'un composé selon la revendication 1 ou 2 comme substrat dans la détermination de l'α-amylase en présence d'α-glucosidase.

7. Utilisation d'un composé selon la formule générale

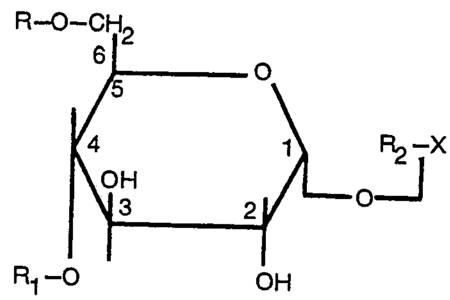

dans laquelle R et $R_1$, indépendamment l'un de l'autre, représentent chacun un groupe alcoyle ou alcanoyle, rectiligne ou ramifié, avec 1 à 6 atomes de carbone ou un radical phényle, R et $R_1$ pouvant également former ensemble un pont méthylène dont les atomes de carbone, indépendamment l'un de l'autre, peuvent être chacun substitué par un groupe alcoyle avec 1 à 5 atomes de carbone ou un groupe phényle, $R_2$ représente un radical oligoglucoside avec 2 à 7 unités glucose et X représente un atome d'hydrogène ou un radical déterminable optiquement, en particulier un groupe nitrophényle, comme substrat dans la détermination de l'αα-amylase en présence d'α-glucosidase.

8. Utilisation d'un composé selon la revendication 2 comme substrat dans la détermination de l'α-amylase en présence d'α-glucosidase.

**Claims for the Contracting States: BE CH LI DE FR GB IT LU NL SE**

1. Compounds of the general formula I

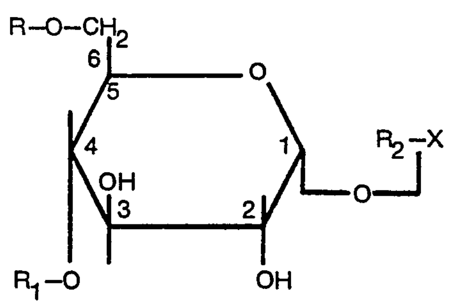

in which R and $R_1$, independently of one another, each represent a straight-chained or branched alkyl or alkoyl group having 1 to 6 carbon atoms or a phenyl radical, wherein R and $R_1$ together can also form a methylene bridge, the hydrogen atoms of which, independently of one another, can be substituted by a respective alkyl group having 1 to 5 carbon atoms or a phenyl group, $R_2$ represents an oligoglucoside residue having 2 to 7 glucose units and X represents a hydrogen atom or a phenyl radical containing a nitro group.

2. A compound according to Claim 1, characterised in that R and $R_1$ together form an ethylidene bridge.

3. A method of preparing a compound according to Claim 1 or 2, characterised in that a compound of the general formula II

$$R_3—X$$

in which X has the meaning given in Claim 1 and $R_3$ is an oligoglucoside residue having 3 to 8 glucose units, is reacted in the presence of p-toluene-sulphonic acid with a compound of the general formula III

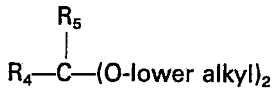

in which $R_4$ and $R_5$, independently of one another, each signify a hydrogen atom or an alkyl group having 1 to 5 carbon atoms or a phenyl group, in a polar organic solvent.

4. A method according to claim 3, characterised in that dimethylformamide is used as the organic solvent.

5. A method according to claim 3 or 4, characterised in that the reaction is carried out between 10°C and 70°C.

6. A method according to any one of Claims 3 to 5, characterised in that the compound of the general formula III is used in excess.

7. Use of a compound according to the general formula

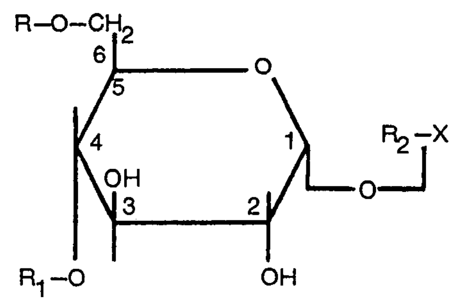

in which R and $R_1$, independently of one another, each represent a straight-chained or branched alkyl or alkoyl group having 1 to 6 carbon atoms or a phenyl radical, wherein R and $R_1$ together can also form a methylene bridge, the hydrogen atoms of which, independently of one another, can be substituted by a respective alkyl group having 1 to 5 carbon atoms or a phenyl group, $R_2$ represents an oligoglucoside residue having 2 to 7 glucose units and X represents a hydrogen atom or an optically determinable residue, in particular a nitro-phenyl group, as a substrate for the determination of α-amylase in the presence of α-glucosidase.

8. Use of a compound according to Claim 2 as a substrate for the determination of α-amylase in the presence of α-glucosidase.

**Claims for the Contracting State: AT**

1. A method of preparing a compound of the general formula I

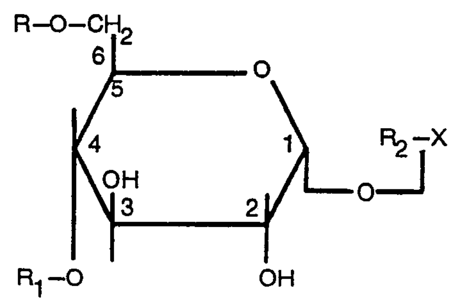

in which R and $R_1$, independently of one another, each represent a straight-chained or branched alkyl or alkoyl group having 1 to 6 carbon atoms or a phenyl radical, wherein R and $R_1$ together can also form a methylene bridge, the hydrogen atoms of which, independently of one another, can be substituted by a respective alkyl group having 1 to 5 carbon atoms or a phenyl group, $R_2$ represents an oligoglucoside residue having 2 to 7 glucose units and X represents a hydrogen atom or a phenyl radical containing a nitro group, characterised in that a compound of the general formula II

$$R_3—X$$

in which X has the meaning given above and $R_3$ is an oligoglucoside residue having 3 to 8 glucose units, is reacted in the presence of p-toluene-sulphonic acid with a compound of the general formula III

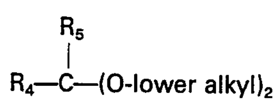

in which $R_4$ and $R_5$, independently of one another, each signify a hydrogen atom or an alkyl group having 1 to 5 carbon atoms or a phenyl group, in a polar organic solvent.

2. A method according to Claim 1, characterised in that R and $R_1$ together form an ethylidene bridge.

3. A method according to Claim 1 or 2, characterised in that dimethylformamide is used as the organic solvent.

4. A method according to Claim 1, 2 or 3, characterised in that the reaction is carried out between 10°C and 70°C.

5. A method according to any one of Claims 1 to 4, characterised in that the compound of the general formula III is used in excess.

6. Use of a compound according to Claim 1 or 2 as a substrate for the determination of α-amylase in the presence of α-glucosidase.

7. Use of a compound according to the general formula

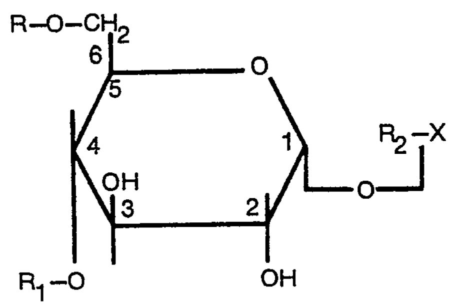

in which R and $R_1$, independently of one another, each represent a straight-chained or branched alkyl or alkoyl group having 1 to 6 carbon atoms or a phenyl radical, wherein R and $R_1$ together can also form a methylene bridge, the hydrogen atoms of which, independently of one another, can be substituted by a respective alkyl group having 1 to 5 carbon atoms or a phenyl group, $R_2$ represents an oligoglucoside residue having 2 to 7 glucose units and X represents a hydrogen atom or an optically determinable residue, in particular a nitro-phenyl group, as a substrate for the determination of α-amylase in the presence of α-glucosidase.

8. Use of a compound according to Claim 2 as a substrate for the determination of α-amylase in the presence of α-glucosidase.